# EUROPEAN PATENT SPECIFICATION

(11) **EP 3 427 650 B1**
(45) Date of publication and mention of the grant of the patent: **23.06.2021**
(21) Application number: 17782510.6
(22) Date of filing: 14.04.2017
(51) Int. Cl.: A61B 5/021, A61B 5/022, A61B 5/08, A61B 5/11, A61B 5/145, A61B 5/00

(54) **BIOLOGICAL INFORMATION ANALYZING DEVICE, SYSTEM, AND PROGRAM**
VORRICHTUNG, SYSTEM UND PROGRAMM ZUR ANALYSE BIOLOGISCHER INFORMATIONEN
DISPOSITIF, SYSTÈME ET PROGRAMME D'ANALYSE D'INFORMATIONS BIOLOGIQUES

(30) Priority: 15.04.2016 JP 2016082463
(43) Date of publication of application: 16.01.2019
(73) Proprietor: Omron Corporation, Kyoto-shi, Kyoto 600-8530 (JP); Omron Healthcare Co., Ltd., Muko-shi, Kyoto 617-0002 (JP)
(72) Inventor: NAKAJIMA, Hiroshi, Kyoto-shi Kyoto 600-8530 (JP); WADA, Hirotaka, Kyoto-shi Kyoto 600-8530 (JP); TSUCHIYA, Naoki, Kyoto-shi Kyoto 600-8530 (JP); KASAI, Masaaki, Kyoto-shi Kyoto 600-8530 (JP); KAN, Eriko, Kyoto-shi Kyoto 600-8530 (JP); UENOYAMA, Toru, Tokyo 108-0075 (JP); OBAYASHI, Keiichi, Kyoto-shi Kyoto 600-8530 (JP); KOKUBO, Ayako, Kyoto-shi Kyoto 600-8530 (JP); OTA, Yuya, Kyoto-shi Kyoto 600-8530 (JP); SHIGA, Toshikazu, Muko-shi Kyoto 617-0002 (JP); KUWABARA, Mitsuo, Muko-shi Kyoto 617-0002 (JP); SATO, Hironori, Muko-shi Kyoto 617-0002 (JP); MIYAGAWA, Ken, Muko-shi Kyoto 617-0002 (JP); TSUTSUMI, Masakazu, Muko-shi Kyoto 617-0002 (JP)
(74) Representative: Isarpatent
(86) International application number: PCT/JP2017/015278
(87) International publication number: WO 2017/179697

(56) References cited:
- WO-A1-99/26529
- DE-A1- 10 243 265
- JP-A- 2014 014 556
- JP-A- 2014 018 272
- JP-A- 2015 100 525
- US-A1- 2015 164 351

## Description

### TECHNICAL FIELD

The present invention relates to a technique for obtaining useful information from a measured blood pressure waveform.

### RELATED ART

Techniques are known in which changes in the internal pressure of the radial artery are measured and the shape of pressure pulses (a blood pressure waveform) is recorded. Patent Document 1 (JP 2008-61824A) discloses measuring a blood pressure waveform through tonometry and obtaining information such as AI (Augmentation Index) value, pulse wave period, baseline fluctuation rate, sharpness, ET (Ejection Time), and the like from the blood pressure waveform. Patent Document 2 (JP 2005-532111A), meanwhile, discloses measuring a blood pressure waveform using a wristwatch-type blood pressure monitor, calculating a mean arterial pressure, a mean systolic pressure, a mean diastolic pressure, a mean systolic pressure index, and a mean diastolic pressure index from the blood pressure waveform, and then outputting an alert when those values deviate from reference values. Patent Document 3 (DE 102 43 265 A1) discloses a bioelectronic cardiac diagnostic measuring device in the form of a glove for optoelectronic sensing of pulse waves with a textile-integrated sensor technology and electronics. Patent Document 4 (JP 2015-100525 A) discloses a diagnostic data generation apparatus including m-dimensional average number-of-vectors calculation means and m+1-dimensional average number-of-vectors calculation means which respectively determine the m-dimensional average number of vectors and the m+1-dimensional average number of vectors, with a distance changed. Patent Document 5 (US 2015/164351 A1) discloses a method that includes obtaining a first data set representing time-varying information on at least one pulse pressure wave within vasculature at the wrist of a subject, obtaining a second data set representing time-varying information about chest vibrations of the subject, and identifying first and second points in the first and second data sets, respectively.

### RELATED ART DOCUMENTS

### PATENT DOCUMENTS

Patent Document 1: JP 2008-61824A
Patent Document 2: JP 2005-532111A
Patent Document 3: DE 102 43 265 A1
Patent Document 4: JP 2015-100525 A
Patent Document 5: US 2015/164351 A1

### SUMMARY OF THE INVENTION

### PROBLEM TO BE SOLVED BY THE INVENTION

The inventors of the present invention are undertaking diligent research toward putting into practical use blood pressure measurement devices capable of accurately measuring a blood pressure waveform for each heartbeat during free movement. Through experiments using test subjects throughout the course of this development, the inventors of the present invention discovered that many types of useful information can be extracted from blood pressure waveform data measured continuously during free movement.

However, it is difficult to intuitively evaluate blood pressure fluctuations, blood pressure abnormalities, and the like simply by reading a time series graph of measured blood pressure waveform data. For example, at a heart rate of 60 BPM, 86,400 blood pressure waveforms will be measured as data over the course of a single day, and finding a waveform of interest (an abnormal waveform or the like) from so many blood pressure waveforms is difficult even for a specialist such as a doctor.

Accordingly, an object of the present invention is to provide a novel technique for intuitively expressing shape characteristics of a blood pressure waveform.

### MEANS FOR SOLVING THE PROBLEMS

To achieve the above-described object, the present invention provides a biological information analyzing device in accordance with claim 1.

The biological information analyzing device according to the present invention includes: an indicator extraction unit configured to create, on the basis of blood pressure waveform time series data measured by a sensor that is configured to be worn on a user's body and non-invasively measure the blood pressure waveform for each of heartbeats, a multidimensional graphic by plotting a plurality of points of data constituting one or more blood pressure waveforms selected from the blood pressure waveform time series data in a multidimensional space constructed from a plurality of axes including at least two axes corresponding to two types of feature amounts extracted from the blood pressure waveform, and extract an indicator pertaining to a shape characteristic of the multidimensional graphic; and a processing unit configured to carry out a process based on the extracted indicator.

Expressing the blood pressure waveform data as a multidimensional graphic in a multidimensional space makes it possible to ascertain an abnormality in the blood pressure waveform as a change in the shape of the multidimensional graphic. Accordingly, abnormalities and the like in the blood pressure waveform can be understood intuitively even by a person lacking specialized knowledge with respect to blood pressure waveforms.

According to the invention, the two types of feature amounts are feature amounts expressing a value changing cyclically with each heartbeat. Selecting two axes in the multidimensional space in this manner results in a closed graphic in which the cross-sectional shape of the multidimensional graphic is substantially elliptical in a plane formed by the two axes. Accordingly, it is easier to obtain the shape characteristics of the multidimensional graphic, and easy to obtain an indicator useful in determining the quality of the blood pressure waveform.

According to the invention, the two types of feature amounts are a blood pressure value and a blood pressure change amount. Preferably, the multidimensional space is a two-dimensional plane, and the multidimensional graphic is a two-dimensional graphic forming a substantially elliptical shape on the two-dimensional plane.

Preferably, the indicator may include at least one of a size of a bounding quadrangle of the two-dimensional graphic, an aspect ratio of a bounding quadrangle of the two-dimensional graphic, a center position of a bounding quadrangle of the two-dimensional graphic, a center of gravity position of the two-dimensional graphic, and an angle of a principle axis of inertia of the two-dimensional graphic. Using such indicators makes it possible to determine the quality of the blood pressure waveform using a simple algorithm.

Preferably, the indicator extraction unit may be configured to extract an indicator for each of blood pressure waveforms for a plurality of heartbeats, and the processing unit may be configured to detect an abnormal blood pressure waveform from the blood pressure waveforms in the plurality of heartbeats by evaluating a similarity of the indicators among the blood pressure waveforms of the plurality of heartbeats. Furthermore, the processing unit preferably may be configured to carry out a process of outputting information pertaining to the detected abnormal blood pressure waveform. Using such a method makes it possible to automatically detect the abnormal blood pressure waveform from multiple blood pressure waveforms. Accordingly, blood pressure abnormalities can be self-checked easily even by a person lacking specialized knowledge with respect to blood pressure waveforms, useful information can be provided to a doctor, a nurse, or the like, and so on.

Preferably, the multidimensional space may be a three-dimensional space, and the multidimensional graphic may be a three-dimensional graphic in which substantially elliptical shapes are distributed throughout the three-dimensional space.

Preferably, of three axes constituting the three-dimensional space, a first axis and a second axis may axes corresponding to feature amounts extracted from the blood pressure waveform, and a third axis is an axis of a factor affecting blood pressure. This makes it possible to evaluate the relationship between a change in the value of the factor and the blood pressure fluctuation.

Preferably, the factor may be temperature. This makes it possible to evaluate the temperature sensitivity of the blood pressure.

Preferably, the indicator may include at least one of a correlation coefficient between a value on the third axis and a center of gravity position of a two-dimensional graphic in a cross-section perpendicular to the third axis of the three-dimensional graphic, a ratio of a change amount of the center of gravity position to a change amount of a value on the third axis, a correlation coefficient between a horizontal width of the two-dimensional graphic and a value on the third axis, and a ratio of a change amount of the horizontal width to a change amount of a value on the third axis. Using such indicators makes it possible to express the relationship between the value of the factor and the blood pressure fluctuation as an indicator, using a simple algorithm.

Preferably, the processing unit may evaluate the strength of a relationship between the user's blood pressure and the factor on the basis of the indicator. Additionally, preferably, the processing unit may carry out a process of outputting information pertaining to the strength of the relationship between the user's blood pressure and the factor. Such a method makes it possible to automatically determine the strength of the relationship between blood pressure and the factor. Accordingly, blood pressure abnormalities can be self-checked easily even by a person lacking specialized knowledge with respect to blood pressure waveforms, useful information can be provided to a doctor, a nurse, or the like, and so on.

The present invention further provides a biological information analyzing method in accordance with claim 13, a program in accordance with claim 12, and a biological information analyzing system in accordance with claim 11.

### EFFECTS OF THE INVENTION

According to the present invention, a novel technique for intuitively expressing shape characteristics of a blood pressure waveform can be provided.

### BRIEF DESCRIPTION OF THE DRAWINGS

Fig. 1 is a diagram illustrating the external appearance of the overall configuration of a biological information analyzing system 10.
Fig. 2 is a block diagram illustrating the hardware configuration of the biological information analyzing system 10.
Fig. 3 is a cross-sectional view schematically illustrating the structure of a blood pressure measurement unit 20 and a measurement state.
Fig. 4 is a diagram illustrating a blood pressure waveform measured by the blood pressure measurement unit 20.
Fig. 5 is a block diagram illustrating processing performed by a biological information analyzing device 1.
Fig. 6 is a diagram illustrating the waveform of a pressure pulse (a blood pressure waveform) in the radial artery, for a single heartbeat.
Fig. 7 is a diagram schematically illustrating a method for generating a post-projection blood pressure trajectory according to Example 1.
Fig. 8 is a flowchart illustrating a process for detecting an abnormality in a blood pressure waveform by using a post-projection blood pressure trajectory, according to Example 1.
Fig. 9 is a diagram illustrating a display example of a detection result according to Example 1.
Fig. 10 is a diagram illustrating another display example of a detection result according to Example 1.
Fig. 11 is a diagram schematically illustrating a method for generating a post-projection blood pressure trajectory according to Example 2.
Figs. 12A and 12B are diagrams illustrating examples of post-projection blood pressure trajectories according to Example 2.
Figs. 13A and 13B are diagrams illustrating examples of post-projection blood pressure trajectories according to Example 2.
Fig. 14 is a diagram illustrating an example of a post-projection blood pressure trajectory according to Example 2.
Fig. 15 is a flowchart illustrating a process for evaluating the temperature sensitivity of blood pressure using a post-projection blood pressure trajectory, according to Example 2.
Fig. 16 is a diagram illustrating a display example of an evaluation result according to Example 2.

### EMBODIMENTS OF THE INVENTION

A preferred embodiment of the present invention will be described below with reference to the drawings. Note, however, that the descriptions of configurations given hereinafter should be changed as appropriate depending on the configuration of the device to which the invention is applied, various types of conditions, and so on, and the scope of the invention is not intended to be limited by the following descriptions.

### Biological Information Analyzing System

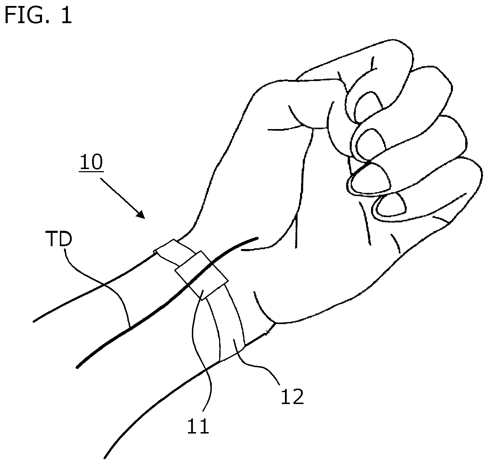

Fig. 1 is a diagram illustrating the external appearance of the overall configuration of a biological information analyzing system 10 according to an embodiment of the present invention. Fig. 1 illustrates a state in which the biological information analyzing system 10 is worn on the left wrist. The biological information analyzing system 10 includes a main unit 11 and a belt 12 fixed to the main unit 11. The biological information analyzing system 10 is what is known as a wearable device, and is worn so that the main unit 11 is in contact with the skin on the inner side of the wrist and so that the main unit 11 is arranged above a radial artery TD located beneath the skin. Although the present embodiment describes a configuration in which the device is worn above the radial artery TD, the configuration may be such that the device is worn above another superficial artery.

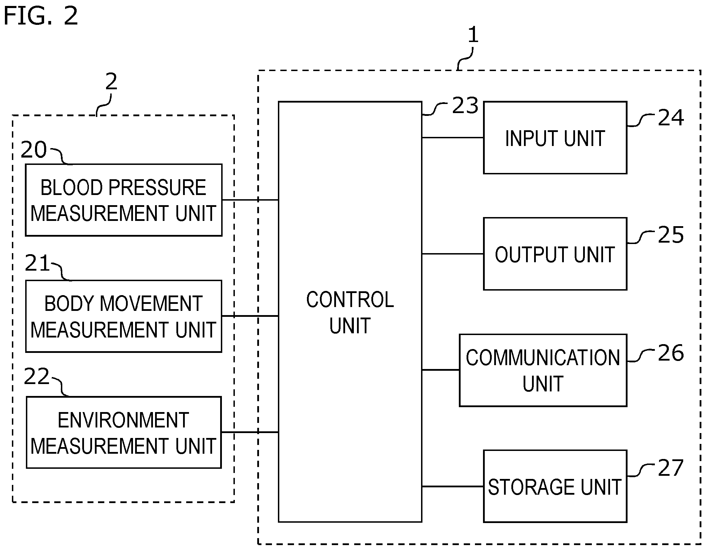

Fig. 2 is a block diagram illustrating the hardware configuration of the biological information analyzing system 10. Broadly speaking, the biological information analyzing system 10 includes a measurement unit 2 and a biological information analyzing device 1. The measurement unit 2 is a device that obtains information used to analyze biological information through measurement, and includes a blood pressure measurement unit 20, a body movement measurement unit 21, and an environment measurement unit 22. However, the configuration of the measurement unit 2 is not limited to that illustrated in Fig. 2. For example, units that measure biological information aside from blood pressure and body movement (body temperature, blood sugar, brain waves, and so on) may be added. Alternatively, units not used in the Example described later are not required configurations and may therefore be omitted from the biological information analyzing system 10. The biological information analyzing device 1 is a device that analyzes biological information on the basis of information obtained from the measurement unit 2, and includes a control unit 23, an input unit 24, an output unit 25, a communication unit 26, and a storage unit 27. The units 20 to 27 are connected to each other by a local bus and other signal lines so as to be capable of exchanging signals. The biological information analyzing system 10 also includes a power source (a battery), which is not illustrated.

The blood pressure measurement unit 20 is a unit that measures pressure pulses in the radial artery TD through tonometry. Tonometry is a non-invasive method of measuring pressure pulses using a pressure sensor, in which an artery is compressed from above the skin at an appropriate pressure to form a flat part in an artery TD, and the internal pressure and external pressure of the artery are balanced.

The body movement measurement unit 21 is a unit, including a three-axis accelerometer, that measures movement of a user's body (body movement) using the accelerometer. The body movement measurement unit 21 may include a circuit that converts the output of the three-axis accelerometer into a format that can be read by the control unit 23.

The environment measurement unit 22 is a unit that measures environment information that can affect the user's physical/mental state (and blood pressure in particular). The environment measurement unit 22 can include a temperature sensor, a humidity sensor, an illuminance sensor, an altitude sensor, a location sensor, and the like, for example. The environment measurement unit 22 may include a circuit that converts the output of these sensors into a format that can be read by the control unit 23.

The control unit 23 is a unit that handles a variety of processes, such as controlling the various parts of the biological information analyzing system 10, acquiring data from the measurement unit 2, storing the acquired data in the storage unit 27, processing/analyzing the data, inputting/outputting the data, and so on. The control unit 23 includes a hardware processor (called a CPU hereinafter), ROM (Read-Only Memory), RAM (Random Access Memory), and the like. The processing carried out by the control unit 23, which will be described later, is realized by the CPU reading programs stored in the ROM or the storage unit 27 and executing those programs. The RAM functions as a work memory when the control unit 23 carries out various types of processes. Although the present embodiment describes a configuration in which the control unit 23 acquires the data from the measurement unit 2 and stores the data in the storage unit 27, the configuration may be such that the data is stored (written) directly from the measurement unit 2 into the storage unit 27.

The constituent elements of the embodiment, e.g., the measurement units, an indicator extraction unit, a processing unit, a determination unit, a risk database, the input unit, the output unit, a case database, and the like may be provided as hardware in the biological information analyzing system 10. The indicator extraction unit, the processing unit, and the determination unit may receive executable programs stored in the storage unit 27 and execute those programs. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as necessary. Databases such as the risk database and the case database may be provided in the storage unit 27 or the like, and may store information arranged so that the data can be searched and accumulated with ease. The structure, operations, and the like of the biological information analyzing system 10 are disclosed in JP 2016-082069, for example. The structure, operations, and the like of the blood pressure measurement unit are disclosed in JP 2016-087003A.

The input unit 24 is a unit that provides an operation interface to the user. For example, operation buttons, switches, a touch panel, or the like can be used.

The output unit 25 is a unit that provides, to the user, an interface that outputs information. For example, a display device that outputs information as images (a liquid crystal display or the like), an audio output device or a buzzer that outputs information as audio, an LED that outputs information by emitting or extinguishing light, a vibration device that outputs information as vibrations, or the like can be used.

The communication unit 26 is a unit that carries out data communication with other devices. Any system, including wireless LAN, Bluetooth (registered trademark), or the like, may be used as the data communication system.

The storage unit 27 is a storage medium in which data can be stored and from which data can be read out, and stores programs executed by the control unit 23, measurement data obtained from the measurement units, various types of data obtained by processing the measurement data, and so on. The storage unit 27 is a medium that electrically, magnetically, optically, mechanically, or chemically stores the information to be stored. Flash memory can be used, for example. The storage unit 27 may be a portable type such as a memory card, or may be built into the biological information analyzing system 10.

Some or all of the body movement measurement unit 21, the environment measurement unit 22, the control unit 23, the input unit 24, the output unit 25, and the storage unit 27 may be configured as devices separate from the main unit 11. In other words, as long as the main unit 11 including the blood pressure measurement unit 20 and a circuit that controls the blood pressure measurement unit 20 can be worn on a wrist, the structures of other units can be designed as desired. In this case, the main unit 11 is linked to the other units via the communication unit 26. A variety of configurations are conceivable, such as implementing the functions of the control unit 23, the input unit 24, the output unit 25, and so on as a smartphone app, or obtaining necessary data from an activity meter having the functions of the body movement measurement unit 21, the environment measurement unit 22, and so on. Sensors that measure biological information aside from blood pressure may be provided as well. For example, a sleep sensor, a pulse oximeter (SpO2 sensor), a breathing sensor (flow sensor), a blood sugar level sensor, and so on may be combined as well.

Although the present embodiment describes providing a sensor that measures blood pressure (the blood pressure measurement unit 20) and a configuration that carries out analysis processes on blood pressure waveform data (the control unit 23 and the like) in a single device, these elements may be configured as separate entities. In the present embodiment, a configuration that carries out analysis processes on biological information (the control unit 23 and the like) is called a "biological information analyzing device", and a device configured by combining a measurement unit and the biological information analyzing device is called a "biological information analyzing system". However, these names are used for the sake of simplicity, and the measurement unit and the configuration that carries out analysis processes on biological information may as a whole be called a "biological information analyzing device", or other names may be used instead.

### Blood Pressure Waveform Measurement

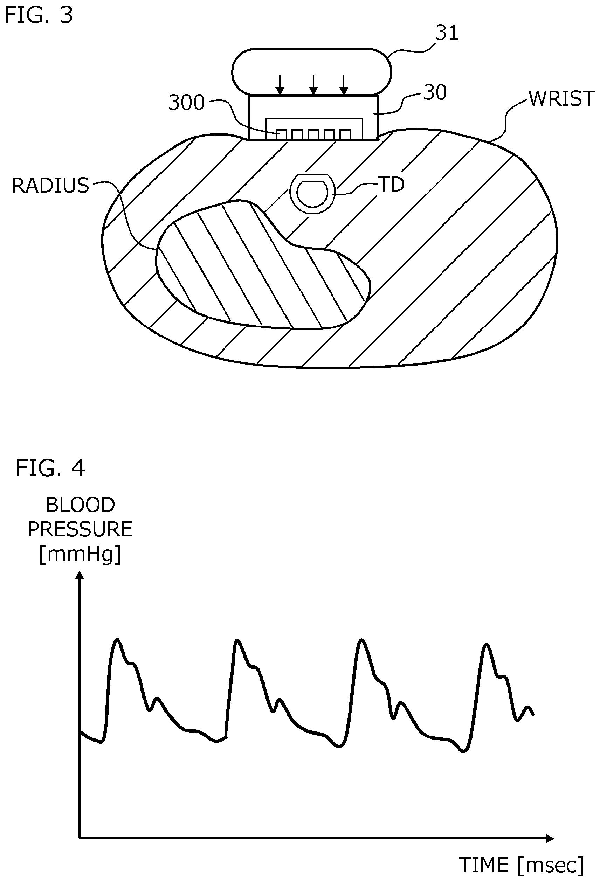

Fig. 3 is a cross-sectional view schematically illustrating the structure of the blood pressure measurement unit 20 and a measurement state. The blood pressure measurement unit 20 includes a pressure sensor 30, and a compression mechanism 31 for pressing the pressure sensor 30 against the wrist. The pressure sensor 30 includes a plurality of pressure detection elements 300. The pressure detection elements 300 are elements that detect a pressure and convert the pressure into an electrical signal, and elements that use a piezoresistance effect, for example, can be favorably used. The compression mechanism 31 is constituted by, for example, an air bladder and a pump that adjusts the internal pressure of the air bladder. When the control unit 23 controls the pump to increase the internal pressure of the air bladder, the air bladder expands and presses the pressure sensor 30 against the surface of the skin. Note that the compression mechanism 31 may be any mechanism capable of adjusting a compressive force of the pressure sensor 30 against the surface of the skin, and is not limited to a mechanism employing an air bladder.

When the biological information analyzing system 10 is secured to the wrist and started, the control unit 23 controls the compression mechanism 31 of the blood pressure measurement unit 20 to keep the compressive force of the pressure sensor 30 in an appropriate state (a tonometry state). Pressure signals detected by the pressure sensor 30 are then acquired sequentially by the control unit 23. The pressure signals obtained by the pressure sensor 30 are generated by taking analog physical amounts (e.g., voltage values) outputted by the pressure detection elements 300 and digitizing those physical amounts through an A/D conversion circuit or the like that employs a known technique. Suitable analog values such as current values, resistance values, or the like may be employed as the analog physical amounts, depending on the types of the pressure detection elements 300. The signal processing such as A/D conversion may be carried out by providing a predetermined circuit in the blood pressure measurement unit 20, or may be carried out by another unit (not shown) provided between the blood pressure measurement unit 20 and the control unit 23. The pressure signals acquired by the control unit 23 correspond to instantaneous values of the internal pressure of the radial artery TD. Accordingly, time series data of a blood pressure waveform can be obtained by acquiring a pressure signal at a time granularity and continuity that enable the blood pressure waveform of a single heartbeat to be obtained. The control unit 23 stores the pressure signals sequentially obtained from the pressure sensor 30 in the storage unit 27 along with information of the measurement times of the signals. The control unit 23 may store the acquired pressure signals as-is in the storage unit 27, or may store the pressure signals in the storage unit 27 after applying necessary signal processing to the pressure signals. The "necessary signal processing" may include processing for correcting the pressure signals so that the amplitude of the pressure signals matches a blood pressure value (e.g., an upper arm blood pressure), processing for reducing or removing noise from the pressure signals, or the like, for example.

Fig. 4 is a diagram illustrating a blood pressure waveform measured by the blood pressure measurement unit 20. The horizontal axis represents time, and the vertical axis represents blood pressure. The sampling frequency can be set as desired, but is preferably set to greater than or equal to 100 Hz in order to reproduce the shape characteristics of the waveform of a single heartbeat. Because the period of a single heartbeat is approximately one second, approximately 100 or more data points can be acquired in the waveform of a single heartbeat.

The blood pressure measurement unit 20 according to the present embodiment has advantages such as those described below.

A blood pressure waveform can be measured for each heartbeat. Thus for example, a variety of indicators related to blood pressure, heart condition, cardiovascular risk, and so on can be obtained on the basis of the shape characteristics of the blood pressure waveform. Additionally, the instantaneous value of the blood pressure can be monitored, which makes it possible to immediately detect blood pressure surges (sudden rises in blood pressure value), reliably detect blood pressure fluctuations and disturbances in the blood pressure waveform appearing only over extremely short amounts of time (one to several heartbeats), and so on.

Blood pressure monitors that are secured to the wrist or upper arm and measure blood pressure through the oscillometric method are in practical use as portable blood pressure monitors. However, a conventional portable blood pressure monitor can only measure a mean blood pressure value from fluctuations in the internal pressure of a cuff over several heartbeats, spanning several seconds to several tens of seconds, and thus cannot obtain time series data of a blood pressure waveform for each heartbeat, as is the case with the blood pressure measurement unit 20 according to the present embodiment.

The blood pressure waveform time series data can be recorded. When the blood pressure waveform time series data is acquired, a variety of indicators pertaining to blood pressure, heart condition, cardiovascular risk, and the like can be obtained by finding characteristics pertaining to changes in the blood pressure waveform over time, analyzing the frequency of the time series data and extracting specific frequency components, and so on, for example.

Because the device is configured as a portable (wearable) device, measurements place little burden on the user, and it is relatively easy to take continuous measurements for long periods of time, monitor blood pressure throughout the entire day, and so on. Furthermore, the portable form makes it possible not only to measure resting blood pressure, but also to measure changes in blood pressure during free movement (e.g., during daily activities, exercise, and so on). This in turn makes it possible to understand the effects of daily activities (sleeping, meals, commuting, work, taking medicine, and so on), exercise, and so on on blood pressure, for example.

Conventional products are devices of a type in which a blood pressure measurement unit is secured to the arm or wrist and the measurement is taken in a state of rest, and changes in blood pressure during daily activities, exercise, and so on cannot be measured, as with the biological information analyzing system 10 according to the present embodiment.

It is easy to link or combine the system with other sensors. For example, causal relationship evaluations or compound evaluations can be made using information obtained from other sensors (body movement, environment information such as temperature, other biological information such as SpO2 or breathing, and the like).

### Biological Information Analyzing Device

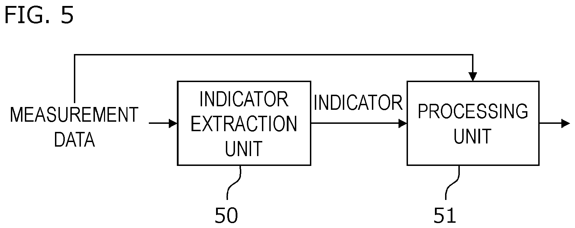

Fig. 5 is a block diagram illustrating processing performed by the biological information analyzing device 1. As illustrated in Fig. 5, the biological information analyzing device 1 includes an indicator extraction unit 50 and a processing unit 51. In the present embodiment, the processes of the indicator extraction unit 50 and the processing unit 51 may be realized by the control unit 23 executing necessary programs. These programs may be stored in the storage unit 27. When executing the necessary programs, the control unit 23 loads the programs in question, which are stored in the ROM or the storage unit 27, into the RAM. The control unit 23 then uses a CPU to interpret and execute the programs loaded into the RAM, and controls the various constituent elements. However, some or all the processing of the indicator extraction unit 50 and the processing unit 51 may be implemented by a circuit such as an ASIC, a FPGA, or the like. Alternatively, some or all of the processing of the indicator extraction unit 50 and the processing unit 51 may be implemented by a computer separate from the main unit 11 (e.g., a smartphone, a tablet terminal, a personal computer, a cloud server, or the like).

The indicator extraction unit 50 obtains, from the storage unit 27, the blood pressure waveform time series data measured continuously by the blood pressure measurement unit 20. The indicator extraction unit 50 extracts an indicator pertaining to characteristics of the blood pressure waveform, from the obtained blood pressure waveform time series data. Here, "characteristics of the blood pressure waveform" include shape characteristics of the blood pressure waveform for a single heartbeat, changes in the blood pressure waveform over time, a frequency component of the blood pressure waveform, and so on. The blood pressure waveform characteristics are not limited thereto, however. The extracted indicator is output to the processing unit 51. Because there are a variety of blood pressure waveform characteristics and indicators, the characteristics and indicators to be extracted can be designed and selected as appropriate in accordance with the purpose of the processing by the processing unit 51. The characteristics and indicators that can be extracted from the blood pressure waveform measurement data in the present embodiment will be described later.

When finding the indicator, the indicator extraction unit 50 can use the measurement data from the body movement measurement unit 21 and/or the measurement data from the environment measurement unit 22 in addition to the blood pressure waveform measurement data. Although not illustrated, measurement data from a sleep sensor, an SpO2 sensor, a breathing sensor (flow sensor), a blood sugar level sensor, or the like may be combined as well. Carrying out a compound analysis on multiple types of measurement data obtained from multiple types of sensors enables a higher level of information analysis to be carried out on the blood pressure waveform. For example, the blood pressure waveform data can be classified into user states, such as resting and active, times of high and low temperature, times of light and deep sleep, breathing and apnea, and so on. Alternatively, causal relationships, correlations, and so on among the measurement data can be evaluated, by extracting the effects of body movement, activity amounts and activity intensity, changes in temperature, how breathing or apnea manifests, and so on on blood pressure.

The processing unit 51 receives the indicator extracted by the indicator extraction unit 50. The processing unit 51 carries out processing on the basis of the received indicator. A variety of processes are conceivable as the processes based on the indicator. For example, the processing unit 51 may provide values of or changes in the extracted indicator to the user, a doctor, a nurse, or the like and used for health management, treatment, health guidance, and so on. Alternatively, the processing unit 51 may predict circulatory system risk, provide guidelines for maintaining one's health or reducing risks, or the like, on the basis of the extracted indicator. Furthermore, if a rise in cardiovascular risk has been detected or predicted on the basis of the indicator, the processing unit 51 may notify the user or an attending doctor, carry out control to prevent activity that will place an excessive burden on the user's heart or prevent the occurrence of the circulatory system event, and so on.

### Information Obtained from Blood Pressure Waveform

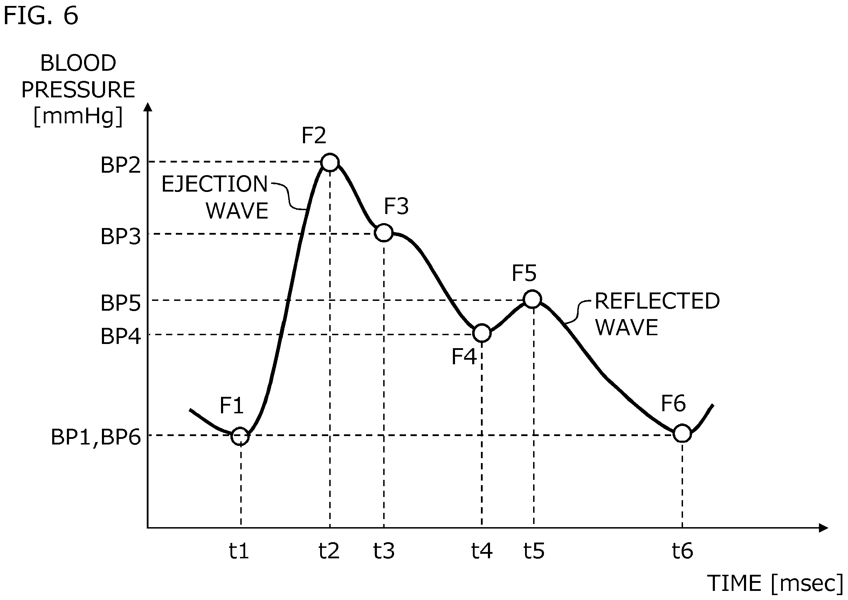

Fig. 6 is a diagram illustrating the waveform of a pressure pulse (a blood pressure waveform) in the radial artery, for a single heartbeat. The horizontal axis represents time t [msec], and the vertical axis represents blood pressure BP [mmHg].

The blood pressure waveform is a compound wave including an "ejection wave" produced when the heart contracts to expel blood and a "reflected wave" produced when the ejection wave is reflected by peripheral vessels, arterial branches, and so on. Examples of characteristic points that can be extracted from a blood pressure waveform corresponding to a single heartbeat are listed below.
- Point F1 is a point corresponding to the rise of the pressure pulse. The point F1 corresponds to an ejection start point of the heart, i.e., a point when the aortic valve opens.
- Point F2 is a point where the amplitude (pressure) of the ejection wave is maximum (a first peak).
- Point F3 is an inflection point appearing partway along the fall of the ejection wave due to the superposition of the reflected wave.
- Point F4 is a minimum point appearing between the ejection wave and the reflected wave, and is also called a "notch". This corresponds to a point when the aortic valve closes.
- Point F5 is a peak in the reflected wave appearing after point F4 (a second peak).
- Point F6 is the end point of the single heartbeat, and corresponds to the ejection start point of the next heartbeat, i.e., the starting point of the next heartbeat.

The indicator extraction unit 50 may use any algorithm to detect the characteristic points. For example, the indicator extraction unit 50 may extract a characteristic point (inflection point) of the blood pressure waveform by operating so as to find a nth-order differential waveforms of the blood pressure waveform and detect a zero crossing point thereof (for points F1, F2, F4, F5, and F6, this can be detected from a first-order differential waveform, and for point F3, from a second-order or fourth-order waveform). Alternatively, the indicator extraction unit 50 may identify the positions of the characteristic points by reading out a waveform pattern, in which characteristic points have been arranged in advance, from the storage unit 27, and fitting the blood pressure waveform in question to the waveform pattern.

By operating on the basis of the times t and the pressures BP of the above characteristic points F1 to F6, the indicator extraction unit 50 can obtain a variety of information (values, feature amounts, indicators, and the like) from the blood pressure waveform of a single heartbeat. The following provides representative examples of information that can be obtained from the blood pressure waveform. Note that tx and BPx represent the time and blood pressure, respectively, of a characteristic point Fx.
- pulse wave interval (heartbeat period) TA = t6 - t1
- heart rate PR = 1/TA
- pulse wave rise time UT = t2 - t1
- systole TS = t4 - t1
- diastole TD = t6 - t4
- reflected wave delay time = t3 - t1
- maximum blood pressure (systolic blood pressure) SBP = BP2
- minimum blood pressure (diastolic blood pressure) DBP = BP 1
- mean blood pressure MAP = area of blood pressure waveform from t1 to t6 / heartbeat period TA
- systolic mean blood pressure = area of blood pressure waveform from t1 to t4 / systole TS
- diastolic mean blood pressure = area of blood pressure waveform from t4 to t6 / diastole TD
- pulse pressure PP = maximum blood pressure SBP - minimum blood pressure DBP
- late systolic pressure SBP2 = BP3
- AI (Augmentation Index) = (late systolic pressure SBP2 - minimum blood pressure DBP) / pulse pressure PP

Basic statistical amounts of this information (values, feature amounts, indicators) can also be used as indicators. The basic statistical amounts include representative values (mean values, median values, mode values, maximum values, minimum values, and the like) and dispersion (variance, standard deviation, coefficient of variation, and the like), for example. Changes over time in this information (values, characteristic values, indicators) can also be used as indicators.

By computing a plurality of pieces of beat information, the indicator extraction unit 50 can obtain an indicator called BRS (baroreflex sensitivity). This is an indicator expressing the capacity of blood pressure to regulate to a constant value. The spontaneous sequence method is an example of the calculation method. This is a method in which only a sequence in which the maximum blood pressure SBP and the pulse wave interval TA rise or fall in synchronization for three or more consecutive beats is extracted, the maximum blood pressure SBP and the pulse wave interval TA are plotted on a two-dimensional plane, and a slope obtained when a regression line is found through the least-squares method is defined as the BRS.

As described thus far, using the biological information analyzing system 10 according to the present embodiment makes it possible to obtain a variety of information from the blood pressure waveform data. However, the biological information analyzing system 10 need not include functions for obtaining all of the above-described information. It is acceptable to provide only the functions for obtaining the necessary information, in accordance with the configuration, user, purpose of usage, location of usage, and so on of the biological information analyzing system 10. Additionally, the configuration may be such that the functions are provided as program modules (application software), and functions can be added by installing the required program modules in the biological information analyzing system 10.

The following Examples describe specific examples of the application of the biological information analyzing system 10.

### Example 1

Using the biological information analyzing system 10 makes it possible to obtain blood pressure waveform data for each heartbeat, which is expected to make it possible to detect blood pressure fluctuations, the occurrence and indications of abnormalities, and so on, which could not be ascertained with conventional periodic blood pressure measurement, ABPM (ambulatory blood pressure monitoring), and so on. However, it is difficult to intuitively evaluate blood pressure fluctuations, abnormalities, and the like simply by reading a time series graph of measured blood pressure waveform data. For example, at a heart rate of 60 BPM, 86,400 blood pressure waveforms will be measured as data over the course of a single day, and finding a waveform of interest (an abnormal waveform or the like) from so many blood pressure waveforms is difficult even for a specialist such as a doctor.

Accordingly, in this Example, the blood pressure waveform of a single heartbeat is converted into a two-dimensional graph in order to intuitively express the shape characteristics of the blood pressure waveform. Specifically, the two-dimensional graph (two-dimensional graphic) is created by plotting a plurality of points of data constituting the blood pressure waveform of a single heartbeat on a two-dimensional plane (two-dimensional space) constructed from two axes corresponding to two types of feature amounts extracted from the blood pressure waveform. In this Example, the two-dimensional graph is called a "post-projection blood pressure trajectory". Anything may be set for the feature amounts corresponding to the axes, but it is preferable that feature amounts having values that change cyclically with each heartbeat be selected. Setting the axes of the two-dimensional plane in this manner results in a trajectory that orbits in a substantially elliptical shape as the post-projection blood pressure trajectory. Using such a two-dimensional graph makes it possible to evaluate, detect, and express abnormalities and the like of a blood pressure waveform with ease, which in turn makes it possible to provide information useful to a doctor, a nurse, or the like. Additionally, abnormalities and the like in the blood pressure waveform can be understood intuitively even by a person lacking specialized knowledge with respect to blood pressure waveforms, which is highly useful for self-checks done by the user, for example.

### Post-Projection Blood Pressure Trajectory

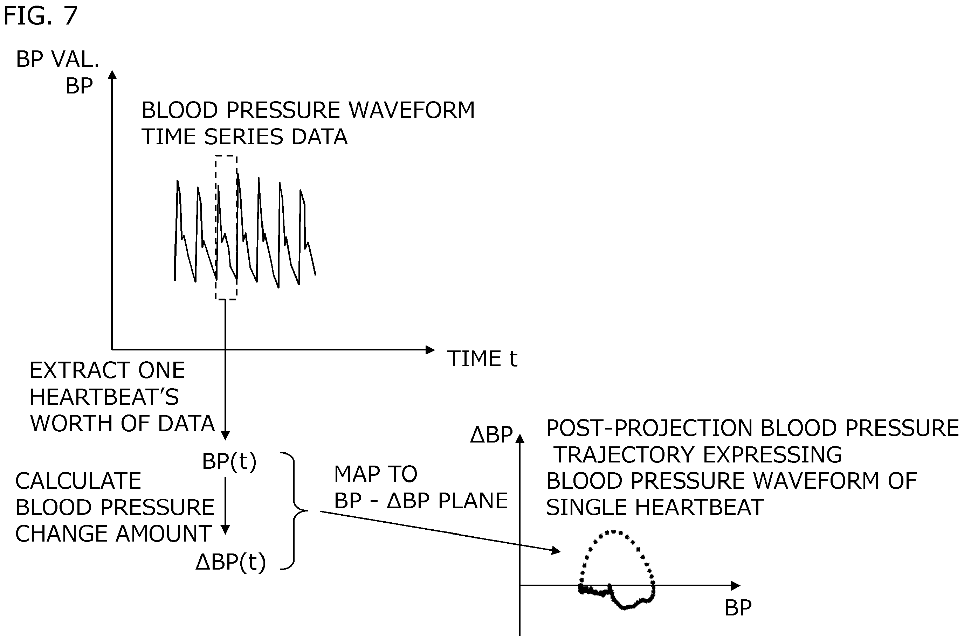

Fig. 7 schematically illustrates a method for generating the post-projection blood pressure trajectory in a two-dimensional graph constructed on the basis of the blood pressure waveform data for a single heartbeat. First, the indicator extraction unit 50 divides the blood pressure waveform time series data into blood pressure waveforms for each heartbeat. For example, the indicator extraction unit 50 may divide the blood pressure waveform on the basis of the position of a diastolic blood pressure (DBP). Blood pressure waveform data BP(t) for a single heartbeat is constituted by a data string of from several tens to several hundreds of points of instantaneous blood pressure values. Assuming that the period of a single heartbeat (a pulse wave interval) is one second and the sampling frequency is 125 Hz, the blood pressure waveform data BP(t) for a single heartbeat is constituted by a data string of 125 points of blood pressure values.

In this Example, a dual-axis two-dimensional plane corresponding to two types of feature amounts, namely a blood pressure value BP and a blood pressure change amount ΔBP, is used to express the blood pressure waveform as a two-dimensional graph. The blood pressure change amount ΔBP is, for example, a difference from the previous instantaneous blood pressure value (ΔBP(t) = BP(t) - BP(t - 1)). Once the indicator extraction unit 50 has mapped all of the data points included in the blood pressure waveform data BP(t) of a single heartbeat onto the two-dimensional plane (the BP - ΔBP plane), the blood pressure waveform corresponding to the single heartbeat is converted into a graphic that traces one cycle's worth of an orbital trajectory, as illustrated in Fig. 7. This graphic is the post-projection blood pressure trajectory. Different blood pressure waveforms produce different shapes in the post-projection blood pressure trajectory. Accordingly, the indicator extraction unit 50 can discover changes, abnormalities, and so on in the blood pressure waveform by evaluating changes, distortion, and so on in the shape of the post-projection blood pressure trajectory. Expressing the blood pressure waveform as a two-dimensional graph results in the complex blood pressure waveform being converted into a simple graphic (a substantially elliptical graphic corresponding to a single cycle), which is advantageous in that it is easy to ascertain changes, abnormalities, and so on in the shape.

### Shape Characteristics of Post-Projection Blood Pressure Trajectory

Preferably, indicators such as those described below are used when evaluating changes, distortion, and so on in the shape of the post-projection blood pressure trajectory.
- size (horizontal width, vertical width, area) of a bounding quadrangle of the post-projection blood pressure trajectory
- aspect ratio and center position of the bounding quadrangle of the post-projection blood pressure trajectory
- center of gravity position of the post-projection blood pressure trajectory
- angle of the principle axis of inertia of the post-projection blood pressure trajectory

For example, a multidimensional vector taking these indicators as elements can be used as a shape feature amount of the post-projection blood pressure trajectory (a shape feature amount vector). If the goal is a simple comparison of the post-projection blood pressure trajectory shape, it is preferable that the post-projection blood pressure trajectories be normalized so the bounding quadrangles are at uniform sizes, angles, and so on, and that the shape feature amounts then be extracted from the normalized post-projection blood pressure trajectories.

### Processing Example

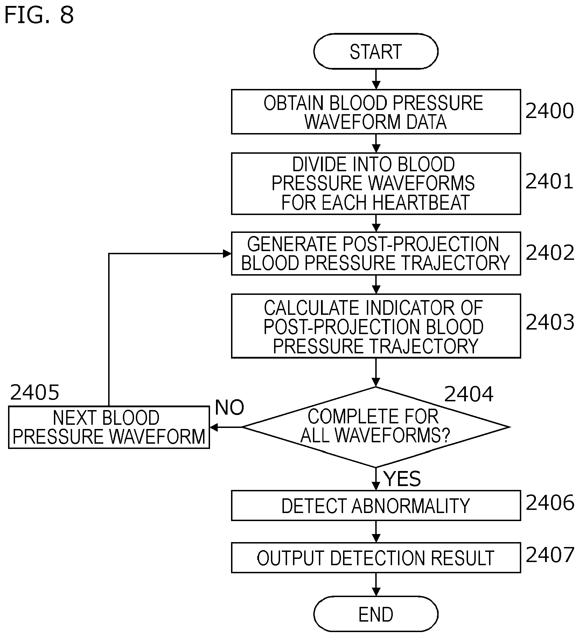

Fig. 8 illustrates an example of a process for detecting an abnormality in the user's blood pressure waveform using the post-projection blood pressure trajectory. First, the indicator extraction unit 50 loads the blood pressure waveform data from the storage unit 27 (step 2400). For example, one day's worth of time series data is loaded. The indicator extraction unit 50 then divides the blood pressure waveform time series data into blood pressure waveforms corresponding to single heartbeats, on the basis of the position of the diastolic blood pressure (DBP) (step 2401).

The indicator extraction unit 50 then generates the post-projection blood pressure trajectory on the basis of the blood pressure waveform data corresponding to a single heartbeat (step 2402). The indicator extraction unit 50 also extracts the indicators pertaining to the shape feature amount of the post-projection blood pressure trajectory (step 2403). The processes of steps 2402 and 2403 are repeated for all of the blood pressure waveforms (steps 2404, 2405).

Next, the processing unit 51 carries out a process for detecting an abnormality in the blood pressure waveform on the basis of the shape feature amount of the post-projection blood pressure trajectory. Specifically, the processing unit 51 detects, from the data of the post-projection blood pressure trajectories for a plurality of heartbeats obtained from steps 2400 to 2405, a post-projection blood pressure trajectory having a shape feature amount significantly different from the others (called an "abnormal post-projection blood pressure trajectory" hereinafter) (step 2406). A known outlier detection algorithm can be applied in the detection of the abnormal post-projection blood pressure trajectory. For example, the processing unit 51 can calculate a degree of similarity between post-projection blood pressure trajectories on the basis of a distance in a feature amount space (a Euclidian distance) and take the trajectory having a total similarity with respect to the other post-projection blood pressure trajectories that is lower than a threshold as the abnormal post-projection blood pressure trajectory. Alternatively, the processing unit 51 may find a distribution of the post-projection blood pressure trajectories in the feature amount space and take a trajectory greater than or equal to a predetermined distance from the center of the distribution (e.g., n times a standard deviation) as the abnormal post-projection blood pressure trajectory.

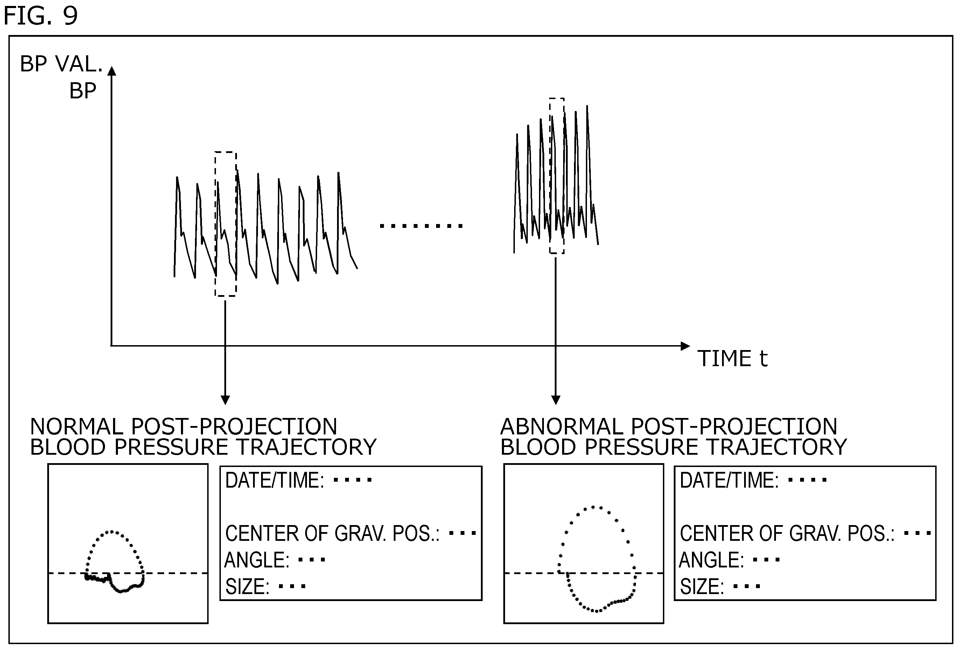

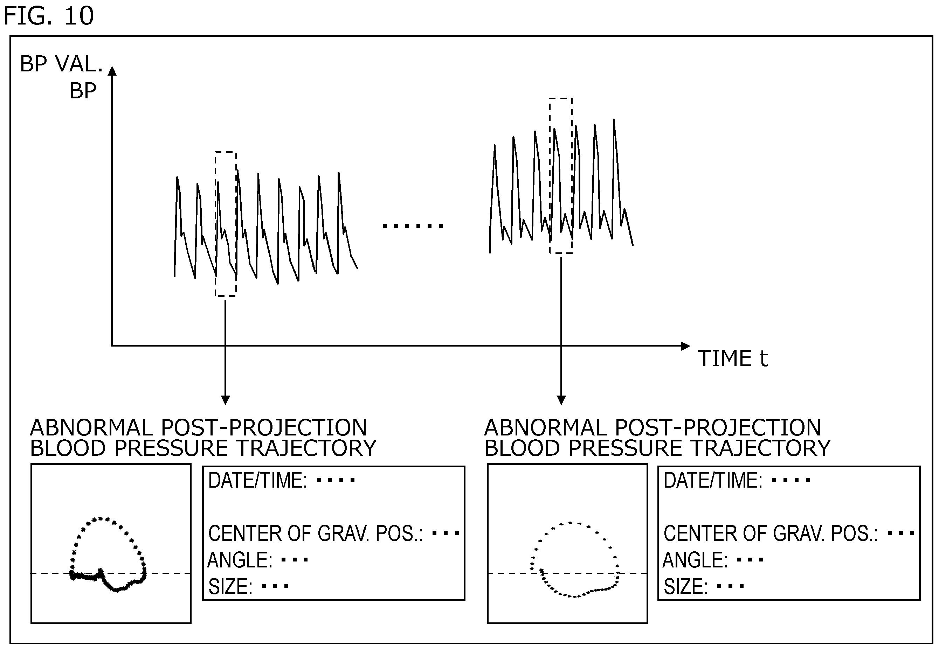

If the abnormal post-projection blood pressure trajectory has been detected, the processing unit 51 outputs information of the detection result to the output unit 25, an external display device, or the like (step 2407). Preferably, the output information includes information pertaining to a blood pressure value for each of units of time, information pertaining to a normal post-projection blood pressure trajectory, information pertaining to the abnormal post-projection blood pressure trajectory, or the like, for example. Fig. 9 is an example of the display of an information output screen for the detection result by the processing unit 51. The normal post-projection blood pressure trajectory and the shape feature amount thereof, the abnormal post-projection blood pressure trajectory and the shape feature amount thereof, and the time at which the abnormality occurred are displayed. Fig. 10 is another example of the display of an information output screen for the detection result by the processing unit 51. The blood pressure waveform time series data, the abnormal post-projection blood pressure trajectories and the shape feature amounts thereof for a plurality of locations, and the times at which the abnormalities occurred are displayed. A doctor, the user, or the like can intuitively ascertain shape changes, abnormalities, and the like in the blood pressure waveform by viewing detection results such as those illustrated in Figs. 9 and 10. The amount of time required to analyze and diagnose the blood pressure time series data during an examination or the like can be made much more efficient as a result. Human error such as overlooking can also be eliminated. Additionally, it is also possible that the disease state can be analyzed in detail.

### Example 2

In medical circles, it is known that rises in blood pressure appear when the ambient temperature (outdoor temperature, indoor temperature, and so on) drops. Conventionally, research has gone no further than evaluating seasonal fluctuations, fluctuations over the course of the day, and so on in blood pressure by totaling blood pressure data acquired through periodic blood pressure measurement, ABPM (ambulatory blood pressure monitoring), and so on. However, if blood pressure waveforms for each heartbeat can be monitored during free movement by using the biological information analyzing system 10, temporary or sudden changes in blood pressure caused by sudden temperature changes (e.g., when the temperature drops suddenly due to inclement weather, when moving from indoors to outdoors, when moving from a heated room to a restroom or a bathroom, and so on) can be observed and evaluated. However, it is difficult to intuitively evaluate blood pressure fluctuations, abnormalities, and the like caused by temperature changes simply by reading a time series graph of measured blood pressure waveform data and temperature data. For example, at a heart rate of 60 BPM, 86,400 blood pressure waveforms will be measured as data over the course of a single day, and discovering the relationship between temperature change and blood pressure fluctuation from so many blood pressure waveforms is difficult even for a specialist such as a doctor.

Accordingly, in this Example, the blood pressure waveform time series data for a plurality of consecutive heartbeats, which expresses a change in the blood pressure waveform, is converted into a three-dimensional graph in order to intuitively indicate a shape feature of the blood pressure waveform, particularly the relationship between blood pressure fluctuation and temperature. Specifically, the three-dimensional graph (three-dimensional graphic) is created by plotting a plurality of points of data constituting the blood pressure waveform of a plurality of heartbeats in a three-dimensional space constructed from two axes, corresponding to two types of feature amounts extracted from the blood pressure waveform, and a temperature axis. In this Example, the three-dimensional graph is called a "post-projection blood pressure trajectory". The post-projection blood pressure trajectory can be said to be a graphic expressing the temperature sensitivity of the blood pressure. Using this method makes it possible to easily evaluate, detect, and express changes in the blood pressure waveform, abnormalities in the blood pressure waveform, and so on with respect to temperature, which in turn makes it possible to provide information useful to a doctor, a nurse, or the like. Additionally, the relationship between temperature and the blood pressure waveform, abnormalities in the blood pressure waveform, and so on can be understood intuitively even by a person lacking specialized knowledge with respect to blood pressure waveforms, which is highly useful for self-checks done by the user, for example.

Although this Example focuses on the effects of temperature changes on blood pressure and therefore uses a three-dimensional space constructed from two axes of feature amounts extracted from the blood pressure waveform and the temperature axis, a factor aside from temperature may be used as the factor that affects blood pressure. For example, combining with measurement data such as body movement, exercise intensity, activity amount, and so on makes it possible to visualize the relationship between changes in attitude (standing or the like) and blood pressure, the relationship between exercise and blood pressure, and so on. In addition, the relationship between apnea and blood pressure may be visualized by combining with measurement data such as SpO2, breathing, or the like. The relationship between drug effects and blood pressure may be visualized by combining with record data of medication dosages, medication taken, and so on. The sensitivity of blood pressure to salt may be visualized by combining with record data of salt intake. Additionally, stress-related hypertension may be visualized by combining with record data of stress levels.

### Post-Projection Blood Pressure Trajectory

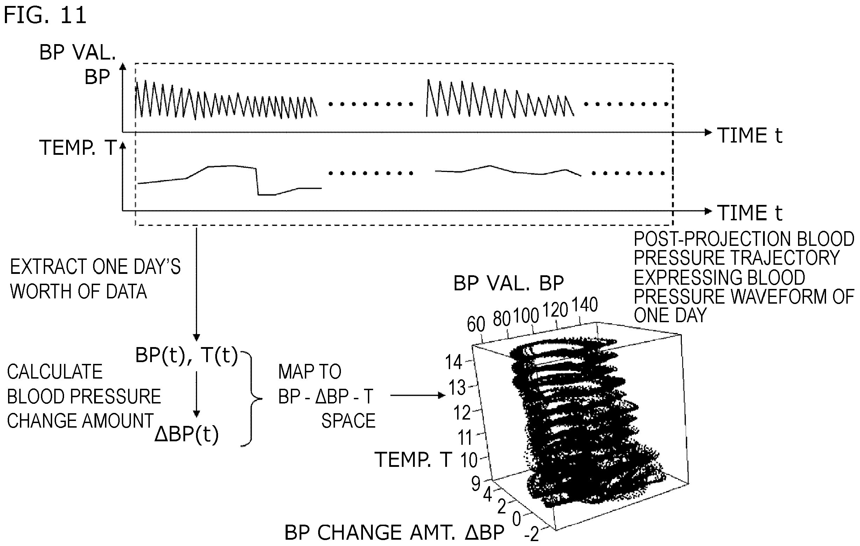

Fig. 11 schematically illustrates a method for generating a three-dimensional graph constructed on the basis of the time series data of blood pressure waveforms for a plurality of consecutive heartbeats, which expresses a change in the blood pressure waveform, and the temperature at the times in the time series data. For example, one day's worth of blood pressure waveform time series data and one day's worth of temperature time series data are used. Preferably, the temperature is the ambient temperature (outdoor temperature, indoor temperature, or the like) of the actual location of the user, measured by the environment measurement unit 22.

One day's worth of the blood pressure waveform data BP(t) is constituted by a data string of instantaneous blood pressure values. Assuming that the sampling frequency of the blood pressure measurement unit 20 is 125 Hz, the one day's worth of the blood pressure waveform data BP(t) is constituted by a data string of approximately 10 million points of blood pressure values. On the other hand, one day's worth of temperature data T(t) is constituted by a data string of instantaneous temperatures. To accurately evaluate the temperature sensitivity of blood pressure, it is preferable that the temperature data T(t) include measurement values for each of the heartbeats (e.g., have a sampling frequency of greater than or equal to 1 Hz).

In this Example, a three-dimensional space constructed from three axes, namely the blood pressure value BP, the blood pressure change amount ΔBP, and temperature T, is used to express the three-dimensional graph of the blood pressure waveform. The blood pressure change amount ΔBP is, for example, a difference from the previous instantaneous blood pressure value (ΔBP(t) = BP(t) - BP(t - 1)). Once the indicator extraction unit 50 has mapped all of the data points included in the blood pressure waveform data BP(t) onto the three-dimensional space (the BP - ΔBP - T space), the blood pressure waveform corresponding to the single heartbeat is converted into a three-dimensional graphic in which an orbital trajectory is traced within the BP - ΔBP plane and a plurality of orbital trajectories are distributed along the temperature T axis, as illustrated in Fig. 11. This graphic is the post-projection blood pressure trajectory. Different blood pressure waveforms produce different shapes in the post-projection blood pressure trajectory. Accordingly, the temperature sensitivity of the blood pressure can be evaluated by evaluating changes in the shape, distortion, and so on in the post-projection blood pressure trajectory along the direction of the temperature T axis. Expressing the blood pressure waveforms as a three-dimensional graph converts massive measurement data of the blood pressure waveforms and temperatures into a simple graphic, which has an advantage in that it is easy to ascertain relationship between shape changes in the post-projection blood pressure trajectory and the temperature, for example.

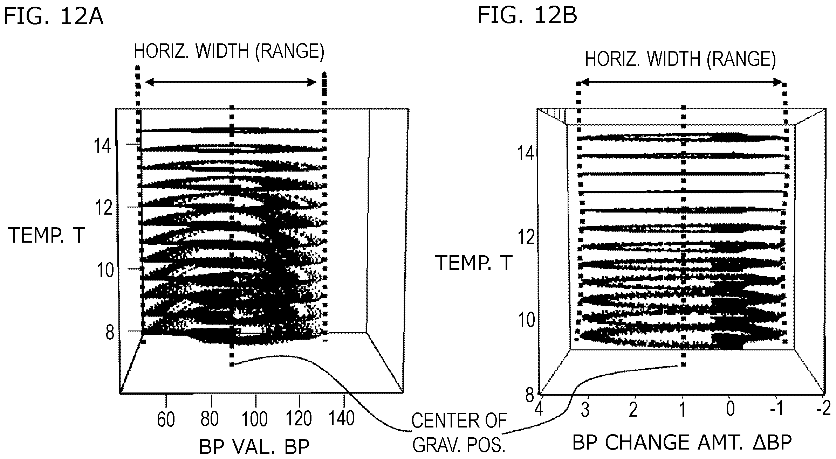

Figs. 12A, 12B, 13A, 13B, and 14 illustrate examples of the post-projection blood pressure trajectory. Figs. 12A and 12B illustrate post-projection blood pressure trajectories generated from the measurement data of a subject 1. Fig. 12A is a view illustrating the relationship between temperature T and the blood pressure value BP, and 12B is a view illustrating the relationship between temperature T and the blood pressure change amount ΔBP. From Figs. 12A and 12B, it can be seen that even if the temperature T changes, the horizontal width (ranges of BP, ΔBP, and so on), the center of gravity position in the BP - ΔBP cross-section, and so on for the post-projection blood pressure trajectories remain almost unchanged. From such shape characteristics, the processing unit 51 can determine that the blood pressure of the subject 1 has no (or low) temperature sensitivity.

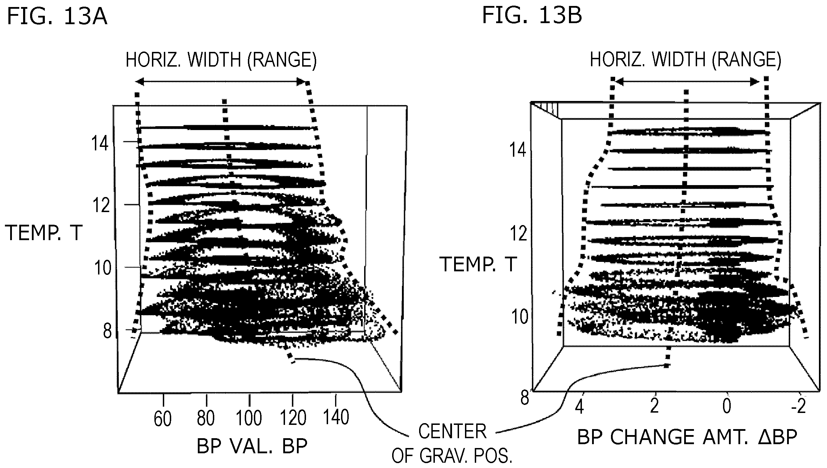

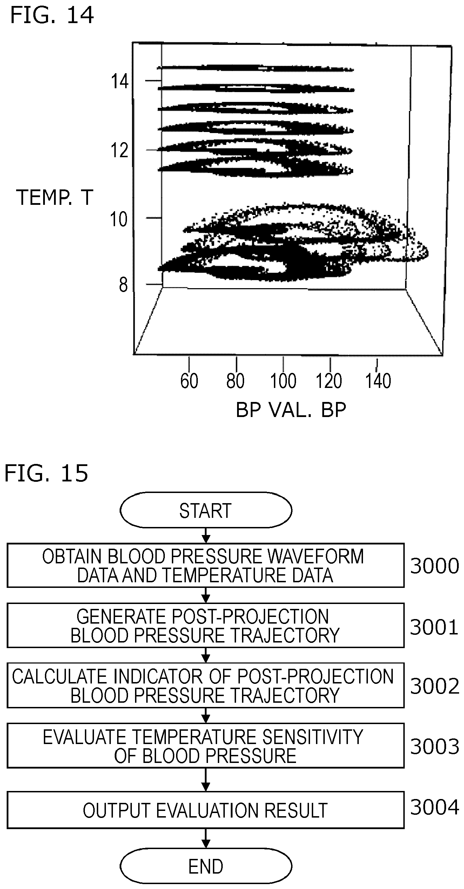

Figs. 13A and 13B illustrate post-projection blood pressure trajectories generated from the measurement data of a subject 2. Fig. 13A is a view illustrating the relationship between temperature T and the blood pressure value BP, and 13B is a view illustrating the relationship between temperature T and the blood pressure change amount ΔBP. From Figs. 13A and 13B, it can be seen that as the temperature T drops, the horizontal width (ranges of BP, ΔBP, and so on) of the post-projection blood pressure trajectories increases, and the center of gravity position in the BP - ΔBP cross-section shifts in a direction in which BP increases. From such shape characteristics, the processing unit 51 can determine that the blood pressure of the subject 2 has temperature sensitivity (or has high temperature sensitivity). Fig. 14 is another example where there is temperature sensitivity. There is a location where the post-projection blood pressure trajectory is not continuous in the direction of the temperature T axis, and the post-projection blood pressure trajectory has a shape disturbance, enlargement, and shifting the center of gravity position at places where the temperature is lower than in that noncontinuous location. This indicates that the blood pressure has been disturbed and has risen due to a sudden drop in temperature. When such shape characteristics have been confirmed, the processing unit 51 can determine that there is temperature sensitivity (or high temperature sensitivity).

### Shape Characteristics of Post-Projection Blood Pressure Trajectory

Preferably, indicators such as those described below are used when evaluating the temperature sensitivity of blood pressure from the post-projection blood pressure trajectory.
- correlation coefficient between center of gravity position C of post-projection blood pressure trajectory in BP - ΔBP cross-section and temperature T
- slope of temperature T relative to center of gravity position C of post-projection blood pressure trajectory in BP - ΔBP cross-section (ratio ΔC/ΔT between change amount ΔT of temperature T and change amount ΔC of center of gravity position C)
- correlation coefficient between center of horizontal width W of post-projection blood pressure trajectory in BP - ΔBP cross-section and temperature T
- slope of temperature T relative to width W of post-projection blood pressure trajectory in BP - ΔBP cross-section (ratio ΔW/ΔT between change amount ΔT of temperature T and change amount ΔW of width W)

It can be said that as the correlation coefficient increases and the slope increases, the temperature sensitivity of the blood pressure is higher.

### Processing Example

Fig. 15 illustrates an example of processing according to this Example. First, the indicator extraction unit 50 loads the blood pressure waveform data and the temperature data from the storage unit 27 (step 3000). For example, one day's worth of blood pressure waveform time series data and one day's worth of temperature time series data are loaded. The indicator extraction unit 50 then generates the post-projection blood pressure trajectory on the basis of the blood pressure waveform data and the temperature data (step 3001). Next, the indicator extraction unit 50 finds indicators such as the above-described correlation coefficient, slope, and so on from the generated post-projection blood pressure trajectory (step 3002).

Next, the processing unit 51 evaluates the temperature sensitivity of the user's blood pressure (the strength of the relationship between the blood pressure and the temperature) on the basis of the indicators found in step 3002 (step 3003). For example, the processing unit 51 may determine the user's temperature sensitivity level by referring to a table in which post-projection blood pressure trajectory indicators and temperature sensitivity levels are associated with each other. Alternatively, the processing unit 51 may determine the user's temperature sensitivity level by referring to a database (table) in which the post-projection blood pressure trajectories and temperature sensitivity levels for multiple subjects are registered and extract data of a subject having attributes (sex, age, and so on) and post-projection blood pressure trajectory indicators similar to those of the user. The table, database, and so on used to determine the temperature sensitivity level may be stored in the storage unit 27 in advance, or may be stored in external storage (a cloud server, for example).

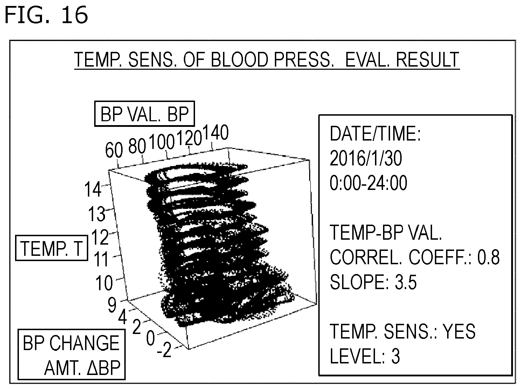

The processing unit 51 outputs information of the evaluation result to the output unit 25, an external display device, or the like (step 3004). Preferably, the output information includes information pertaining to the post-projection blood pressure trajectory, information pertaining to the temperature sensitivity of the blood pressure, and so on, for example. Fig. 16 is an example of the display of an information output screen for the evaluation result by the processing unit 51. The post-projection blood pressure trajectory shape, the post-projection blood pressure trajectory indicators, the temperature sensitivity level, and so on are displayed. A doctor, the user, or the like can intuitively and convincingly understand the temperature sensitivity of the blood pressure by viewing such evaluation results.

Note that the configurations in the above-described embodiment and Example are merely specific examples of the present invention, and are not intended to limit the scope of the present invention. The present invention can employ a variety of specific configurations without departing from the scope of the invention defined by the claims. For example, although Example 1 describes mapping the blood pressure waveform of a single heartbeat onto a two-dimensional space, a multidimensional graph may be generated by mapping data onto a multidimensional space constructed from three or more axes. In Example 2, too, the data may be mapped onto a multidimensional space constructed from four or more axes.

### INDEX TO THE REFERENCE NUMERALS

- 1: biological information analyzing device
- 2: measurement unit
- 10: biological information analyzing system
- 11: main unit
- 12: belt
- 20: blood pressure measurement unit
- 21: body movement measurement unit
- 22: environment measurement unit
- 23: control unit
- 24: input unit
- 25: output unit
- 26: communication unit
- 27: storage unit
- 30: pressure sensor
- 31: compression mechanism
- 300: pressure detection element
- 50: indicator extraction unit
- 51: processing unit

## Claims

1. A biological information analyzing device (1) comprising:
an indicator extraction unit (50) configured to create, on the basis of blood pressure waveform time series data measured by a sensor (30) that is configured to be worn on a user's body and non-invasively measure the blood pressure waveform for each of heartbeats, a multidimensional graphic by plotting a plurality of points of data constituting one or more blood pressure waveforms selected from the blood pressure waveform time series data in a multidimensional space constructed from a plurality of axes including at least two axes corresponding to two types of feature amounts extracted from the blood pressure waveform, and extract an indicator pertaining to a shape characteristic of the multidimensional graphic; and
a processing unit (51) configured to carry out a process based on the extracted indicator,
wherein the two types of feature amounts are feature amounts expressing a value changing cyclically with each heartbeat, and
wherein the two types of feature amounts are a blood pressure value and a blood pressure change amount.

2. The biological information analyzing device (1) according to claim 1,
wherein the multidimensional space is a two-dimensional plane; and
the multidimensional graphic is a closed graphic forming a substantially elliptical shape on the two-dimensional plane.

3. The biological information analyzing device (1) according to claim 2,
wherein the indicator includes at least one of a size of a bounding quadrangle of the two-dimensional graphic, an aspect ratio of a bounding quadrangle of the two-dimensional graphic, a center position of a bounding quadrangle of the two-dimensional graphic, a center of gravity position of the two-dimensional graphic, and an angle of a principle axis of inertia of the two-dimensional graphic.

4. The biological information analyzing device (1) according to any one of claims 1 to 3,
wherein the indicator extraction unit (50) is configured to extract an indicator for each of blood pressure waveforms in a plurality of heartbeats; and
the processing unit (51) is configured to detect an abnormal blood pressure waveform from the blood pressure waveforms in the plurality of heartbeats by evaluating a similarity of the indicators among the blood pressure waveforms of the plurality of heartbeats.

5. The biological information analyzing device (1) according to claim 4,
wherein the processing unit (51) is configured to carry out a process of outputting information pertaining to the detected abnormal blood pressure waveform.

6. The biological information analyzing device (1) according to claim 1,
wherein the multidimensional space is a three-dimensional space; and
the multidimensional graphic is a three-dimensional graphic in which closed graphics forming substantially elliptical shapes are distributed throughout the three-dimensional space.

7. The biological information analyzing device (1) according to claim 6,
wherein of three axes constituting the three-dimensional space, a first axis and a second axis are axes corresponding to feature amounts extracted from the blood pressure waveform, and a third axis is an axis of a factor affecting blood pressure.

8. The biological information analyzing device (1) according to claim 7,
wherein the factor is temperature.

9. The biological information analyzing device (1) according to claim 7 or 8,
wherein the indicator includes at least one of a correlation coefficient between a value on the third axis and a center of gravity position of a two-dimensional graphic in a cross-section perpendicular to the third axis of the three-dimensional graphic, a ratio of a change amount of the center of gravity position to a change amount of a value on the third axis, a correlation coefficient between a horizontal width of the two-dimensional graphic and a value on the third axis, and a ratio of a change amount of the horizontal width to a change amount of a value on the third axis.

10. The biological information analyzing device (1) according to any one of claims 7 to 9,
wherein the processing unit (51) is configured to evaluate the strength of a relationship between the user's blood pressure and the factor on the basis of the indicator.

11. A biological information analyzing system (10) comprising:
a sensor (30), which is configured to be worn on a user's body and non-invasively measure a blood pressure waveform for each of heartbeats; and
the biological information analyzing device (1) according to any one of claims 1 to 10, the biological information analyzing device (1) being configured to analyze biological information using data of the blood pressure waveform measured continuously by the sensor (30).

12. A program causing a processor to function as the indicator extraction unit (50) and the processing unit (51) of the biological information analyzing device (1) according to any one of claims 1 to 10.

13. A biological information analyzing method comprising:
a step of creating, by means of an indicator extraction unit (50), on the basis of blood pressure waveform time series data measured by a sensor (30) that is worn on a user's body and can non-invasively measure the blood pressure waveform for each of heartbeats, a multidimensional graphic by plotting a plurality of points of data constituting one or more blood pressure waveforms selected from the blood pressure waveform time series data in a multidimensional space constructed from a plurality of axes including at least two axes corresponding to two types of feature amounts extracted from the blood pressure waveform, and extracting an indicator pertaining to a shape characteristic of the multidimensional graphic; and
a step of carrying out a process based on the extracted indicator by means of a processing unit (51),
wherein the two types of feature amounts are feature amounts expressing a value changing cyclically with each heartbeat, and
wherein the two types of feature amounts are a blood pressure value and a blood pressure change amount.

## Patentansprüche

1. Vorrichtung (1) zur Analyse biologischer Informationen, umfassend:
eine Indikatorextraktionseinheit (50), die dafür eingerichtet ist, auf der Basis von Blutdruckwellenform-Zeitreihendaten, die durch einen Sensor (30) gemessen werden, der dafür eingerichtet ist, am Körper eines Benutzers getragen zu werden und die Blutdruckwellenform für jeden einzelnen Herzschlag auf nicht-invasive Weise zu messen, eine mehrdimensionale Grafik zu erstellen, indem mehrere Punkte von Daten, die eine oder mehrere Blutdruckwellenformen darstellen, die aus den Blutdruckwellenform-Zeitreihendaten ausgewählt sind, in einem mehrdimensionalen Raum aufgetragen werden, der aus mehreren Achsen gebildet ist, die mindestens zwei Achsen umfassen, die zwei Arten von Merkmalsmengen entsprechen, die aus der Blutdruckwellenform extrahiert wurden, sowie einen Indikator zu extrahieren, der sich auf eine Formcharakteristik der mehrdimensionalen Grafik bezieht; und
eine Verarbeitungseinheit (51), die dafür eingerichtet ist, einen Prozess auf der Grundlage des extrahierten Indikators auszuführen,
wobei die beiden Arten von Merkmalsmengen Merkmalsmengen sind, die einen Wert ausdrücken, der sich zyklisch mit jedem Herzschlag ändert, und
wobei die beiden Arten von Merkmalsmengen ein Blutdruckwert und ein Blutdruckänderungsbetrag sind.

2. Vorrichtung (1) zur Analyse biologischer Informationen nach Anspruch 1, wobei
der mehrdimensionale Raum eine zweidimensionale Ebene ist; und
die mehrdimensionale Grafik eine geschlossene Grafik ist, die eine im Wesentlichen elliptische Form auf der zweidimensionalen Ebene bildet.

3. Vorrichtung (1) zur Analyse biologischer Informationen nach Anspruch 2, wobei der Indikator mindestens eines von einer Größe eines Begrenzungsvierecks der zweidimensionalen Grafik, einem Seitenverhältnis eines Begrenzungsvierecks der zweidimensionalen Grafik, einer mittigen Position eines Begrenzungsvierecks der zweidimensionalen Grafik, einer Schwerpunktposition der zweidimensionalen Grafik und einem Winkel einer Hauptträgheitsachse der zweidimensionalen Grafik umfasst.

4. Vorrichtung (1) zur Analyse biologischer Informationen nach einem der Ansprüche 1 bis 3, wobei
die Indikatorextraktionseinheit (50) dafür eingerichtet ist, einen Indikator für jede Blutdruckwellenform in mehreren Herzschlägen zu extrahieren; und
die Verarbeitungseinheit (51) dafür eingerichtet ist, eine abnormale Blutdruckwellenform aus den Blutdruckwellenformen in den mehreren Herzschlägen durch Auswerten einer Ähnlichkeit der Indikatoren unter den Blutdruckwellenformen der mehreren Herzschläge zu erkennen.

5. Vorrichtung (1) zur Analyse biologischer Informationen nach Anspruch 4, wobei die Verarbeitungseinheit (51) dafür eingerichtet ist, einen Prozess zum Ausgeben von Informationen bezüglich der detektierten abnormalen Blutdruckwellenform auszuführen.

6. Vorrichtung (1) zur Analyse biologischer Informationen nach Anspruch 1, wobei
der mehrdimensionale Raum ein dreidimensionaler Raum ist; und
die mehrdimensionale Grafik eine dreidimensionale Grafik ist, in der geschlossene Grafiken, die im Wesentlichen elliptische Formen bilden, über den gesamten dreidimensionalen Raum verteilt sind.

7. Vorrichtung (1) zur Analyse biologischer Informationen nach Anspruch 6, wobei von drei Achsen, die den dreidimensionalen Raum bilden, eine erste Achse und eine zweite Achse Achsen sind, die Merkmalsmengen entsprechen, die aus der Blutdruckwellenform extrahiert wurden, und eine dritte Achse eine Achse eines Faktors ist, der den Blutdruck beeinflusst.

8. Vorrichtung (1) zur Analyse biologischer Informationen nach Anspruch 7, wobei der Faktor eine Temperatur ist.

9. Vorrichtung (1) zur Analyse biologischer Informationen nach Anspruch 7 oder 8, wobei der Indikator mindestens eines von einem Korrelationskoeffizienten zwischen einem Wert auf der dritten Achse und einer Schwerpunktposition einer zweidimensionalen Grafik in einem Querschnitt senkrecht zu der dritten Achse der dreidimensionalen Grafik, einem Verhältnis eines Änderungsbetrages der Schwerpunktposition zu einem Änderungsbetrag eines Wertes auf der dritten Achse, einem Korrelationskoeffizienten zwischen einer horizontalen Breite der zweidimensionalen Grafik und einem Wert auf der dritten Achse und einem Verhältnis eines Änderungsbetrages der horizontalen Breite zu einem Änderungsbetrag eines Wertes auf der dritten Achse umfasst.

10. Vorrichtung (1) zur Analyse biologischer Informationen nach einem der Ansprüche 7 bis 9, wobei die Verarbeitungseinheit (51) dafür eingerichtet ist, die Stärke einer Beziehung zwischen dem Blutdruck des Benutzers und dem Faktor auf der Grundlage des Indikators zu bewerten.

11. System (10) zur Analyse biologischer Informationen, das umfasst:
einen Sensor (30), der dafür eingerichtet ist, am Körper des Benutzers getragen zu werden und auf nicht-invasive Weise eine Blutdruckwellenform für jeden einzelnen Herzschlag zu messen; und
die Vorrichtung (1) zur Analyse biologischer Informationen nach einem der Ansprüche 1 bis 10, wobei die Vorrichtung (1) zur Analyse biologischer Informationen dafür eingerichtet ist, biologische Informationen unter Verwendung von Daten der Blutdruckwellenform zu analysieren, die kontinuierlich durch den Sensor (30) gemessen wird.

12. Programm, das einen Prozessor veranlasst, als die Indikatorextraktionseinheit (50) und die Verarbeitungseinheit (51) der Vorrichtung (1) zur Analyse biologischer Informationen nach einem der Ansprüche 1 bis 10 zu dienen.

13. Verfahren zur Analyse biologischer Informationen, umfassend:
einen Schritt zum Erstellen, mittels einer Indikatorextraktionseinheit (50), auf der Basis von Blutdruckwellenform-Zeitreihendaten, die durch einen Sensor (30) gemessen werden, der dafür eingerichtet ist, am Körper eines Benutzers getragen zu werden und die Blutdruckwellenform für jeden einzelnen Herzschlag auf nicht-invasive Weise zu messen, einer mehrdimensionalen Grafik, indem mehrere Punkte von Daten, die eine oder mehrere Blutdruckwellenformen darstellen, die aus den Blutdruckwellenform-Zeitreihendaten ausgewählt sind, in einem mehrdimensionalen Raum aufgetragen werden, der aus mehreren Achsen gebildet ist, die mindestens zwei Achsen umfassen, die zwei Arten von Merkmalsmengen entsprechen, die aus der Blutdruckwellenform extrahiert wurden, sowie zum Extrahieren eines Indikators, der sich auf eine Formcharakteristik der mehrdimensionalen Grafik bezieht; und
einen Schritt zum Ausführen eines Prozesses auf der Grundlage des extrahierten Indikators mittels einer Verarbeitungseinheit (51),
wobei die beiden Arten von Merkmalsmengen Merkmalsmengen sind, die einen Wert ausdrücken, der sich zyklisch mit jedem Herzschlag ändert, und
wobei die beiden Arten von Merkmalsmengen ein Blutdruckwert und ein Blutdruckänderungsbetrag sind.

## Revendications

1. Dispositif d'analyse d'informations biologiques (1) comprenant :
une unité d'extraction d'indicateur (50) configurée pour créer, sur la base de données de série temporelle de forme d'onde de pression sanguine mesurées par un capteur (30) qui est configuré pour être porté sur le corps d'un utilisateur et mesurer de manière non invasive la forme d'onde de pression sanguine pour chacun de battements cardiaques, un graphique multidimensionnel par traçage d'une pluralité de points de données constituant une ou plusieurs formes d'onde de pression sanguine sélectionnées à partir des données de série temporelle de forme d'onde de pression sanguine dans un espace multidimensionnel construit à partir d'une pluralité d'axes comportant au moins deux axes correspondant à deux types de quantités de particularité extraites de la forme d'onde de pression sanguine, et extraire un indicateur concernant une caractéristique de forme du graphique multidimensionnel ; et
une unité de traitement (51) configurée pour exécuter un processus d'après l'indicateur extrait,
dans lequel les deux types de quantités de particularité sont des quantités de particularité exprimant une valeur qui change cycliquement avec chaque battement cardiaque, et
dans lequel les deux types de quantités de particularité sont une valeur de pression sanguine et une quantité de changement de pression sanguine.

2. Dispositif d'analyse d'informations biologiques (1) selon la revendication 1,
dans lequel l'espace multidimensionnel est un plan bidimensionnel ; et
le graphique multidimensionnel est un graphique fermé formant une forme sensiblement elliptique sur le plan bidimensionnel.

3. Dispositif d'analyse d'informations biologiques (1) selon la revendication 2,
dans lequel l'indicateur comporte au moins l'un parmi une taille d'un rectangle englobant du graphique bidimensionnel, un facteur de forme d'un rectangle englobant du graphique bidimensionnel, une position de centre d'un rectangle englobant du graphique bidimensionnel, une position de centre de gravité du graphique bidimensionnel, et un angle d'un axe d'inertie de principe du graphique bidimensionnel.

4. Dispositif d'analyse d'informations biologiques (1) selon l'une quelconque des revendications 1 à 3,
dans lequel l'unité d'extraction d'indicateur (50) est configurée pour extraire un indicateur pour chacune de formes d'onde de pression sanguine dans une pluralité de battements cardiaques ; et
l'unité de traitement (51) est configurée pour détecter une forme d'onde de pression sanguine anormale parmi les formes d'onde de pression sanguine dans la pluralité de battements cardiaques par l'évaluation d'une similitude des indicateurs parmi les formes d'onde de pression sanguine de la pluralité de battements cardiaques.

5. Dispositif d'analyse d'informations biologiques (1) selon la revendication 4,
dans lequel l'unité de traitement (51) est configurée pour exécuter un processus de fourniture en sortie d'informations concernant la forme d'onde de pression sanguine anormale détectée.

6. Dispositif d'analyse d'informations biologiques (1) selon la revendication 1,
dans lequel l'espace multidimensionnel est un espace tridimensionnel ; et
le graphique multidimensionnel est un graphique tridimensionnel dans lequel des graphiques fermés formant des formes sensiblement elliptiques sont répartis dans tout l'espace tridimensionnel.

7. Dispositif d'analyse d'informations biologiques (1) selon la revendication 6,
dans lequel des trois axes constituant l'espace tridimensionnel, un premier axe et un deuxième axe sont des axes correspondant à des quantités de particularité extraites de la forme d'onde de pression sanguine, et un troisième axe est un axe d'un facteur influençant la pression sanguine.

8. Dispositif d'analyse d'informations biologiques (1) selon la revendication 7,
dans lequel le facteur est une température.

9. Dispositif d'analyse d'informations biologiques (1) selon la revendication 7 ou 8,
dans lequel l'indicateur comporte au moins l'un parmi un coefficient de corrélation entre une valeur sur le troisième axe et une position de centre de gravité d'un graphique bidimensionnel dans une section transversale perpendiculaire au troisième axe du graphique tridimensionnel, un rapport entre une quantité de changement de la position de centre de gravité et une quantité de changement d'une valeur sur le troisième axe, un coefficient de corrélation entre une largeur horizontale du graphique bidimensionnel et une valeur sur le troisième axe, et un rapport entre une quantité de changement de la largeur horizontale et une quantité de changement d'une valeur sur le troisième axe.

10. Dispositif d'analyse d'informations biologiques (1) selon l'une quelconque des revendications 7 à 9,
dans lequel l'unité de traitement (51) est configurée pour évaluer la force d'une relation entre la pression sanguine de l'utilisateur et le facteur sur la base de l'indicateur.

11. Système d'analyse d'informations biologiques (10) comprenant :
un capteur (30), qui est configuré pour être porté sur le corps d'un utilisateur et mesurer de manière non invasive une forme d'onde de pression sanguine pour chacun de battements cardiaques ; et
le dispositif d'analyse d'informations biologiques (1) selon l'une quelconque des revendications 1 à 10, le dispositif d'analyse d'informations biologiques (1) étant configuré pour analyser des informations biologiques à l'aide de données de la forme d'onde de pression sanguine mesurées en continu par le capteur (30).

12. Programme amenant un processeur à fonctionner en tant qu'unité d'extraction d'indicateur (50) et unité de traitement (51) du dispositif d'analyse d'informations biologiques (1) selon l'une quelconque des revendications 1 à 10.

13. Procédé d'analyse d'informations biologiques comprenant :
une étape consistant à créer, au moyen d'une unité d'extraction d'indicateur (50), sur la base de données de série temporelle de forme d'onde de pression sanguine mesurées par un capteur (30) qui est porté sur le corps d'un utilisateur et peut mesurer de manière non invasive la forme d'onde de pression sanguine pour chacun de battements cardiaques, un graphique multidimensionnel par traçage d'une pluralité de points de données constituant une ou plusieurs formes d'onde de pression sanguine sélectionnées à partir des données de série temporelle de forme d'onde de pression sanguine dans un espace multidimensionnel construit à partir d'une pluralité d'axes comportant au moins deux axes correspondant à deux types de quantités de particularité extraites de la forme d'onde de pression sanguine, et extraire un indicateur concernant une caractéristique de forme du graphique multidimensionnel ; et
une étape consistant à exécuter un processus d'après l'indicateur extrait au moyen d'une unité de traitement (51),
dans lequel les deux types de quantités de particularité sont des quantités de particularité exprimant une valeur qui change cycliquement avec chaque battement cardiaque, et dans lequel les deux types de quantités de particularité sont une valeur de pression sanguine et une quantité de changement de pression sanguine.
